(19) Europäisches Patentamt
European Patent Office
Office européen des brevets

(11) Numéro de publication : **0 320 339 B1**

# (12) FASCICULE DE BREVET EUROPEEN

(45) Date de publication du fascicule du brevet : **18.03.92 Bulletin 92/12**

(51) Int. Cl.$^5$ : **C07C 29/136,** C07C 29/14, C07C 33/02

(21) Numéro de dépôt : **88403015.6**

(22) Date de dépôt : **30.11.88**

## (54) Procédé de préparation d'alcools insaturés.

(30) Priorité : **01.12.87 FR 8716627**

(43) Date de publication de la demande : **14.06.89 Bulletin 89/24**

(45) Mention de la délivrance du brevet : **18.03.92 Bulletin 92/12**

(84) Etats contractants désignés : **AT BE CH DE ES FR GB GR IT LI LU NL SE**

(56) Documents cités :
**EP-A- 0 024 648**
**EP-A- 0 061 337**
**US-A- 3 968 147**

(73) Titulaire : **RHONE-POULENC SANTE**
**20, avenue Raymond Aron**
**F-92160 Antony (FR)**

(72) Inventeur : **Grosselin, Jean-Michel**
**15 rue Terraille**
**F-69001 Lyon (FR)**
Inventeur : **Mercier, Claude**
**85 avenue du Point du Jour**
**F-69005 Lyon (FR)**

(74) Mandataire : **Le Pennec, Magali et al**
**RHONE-POULENC RORER SA, Direction des Brevets, 20 Avenue Raymond Aron**
**F-92165 Antony Cédex (FR)**

EP 0 320 339 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation d'alcools insaturés primaires ou secondaires par hydrogénation des aldéhydes ou des cétones correspondants.

En effet, l'objet de la présente invention concerne un procédé pour la préparation des alcools insaturés de formule générale :

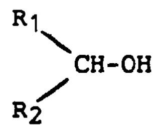
(I)

dans laquelle $R_1$ et $R_2$, identiques ou différents, représentent un atome d'hydrogène ou un radical aliphatique saturé ou non saturé, éventuellement substitué par un radical alicyclique saturé ou non saturé ou par un radical aromatique, ou un radical alicyclique saturé ou non saturé ou un radical aromatique, ou bien $R_1$ et $R_2$ forment ensemble un radical alicyclique non saturé, étant entendu que :

- $R_1$ et $R_2$ ne peuvent pas représenter simultanément un atome d'hydrogène,
- l'un au moins des radicaux $R_1$ ou $R_2$ contient une double liaison éthylénique,
- les radicaux aliphatiques, alicycliques ou aromatiques peuvent être éventuellement substitués par un ou plusieurs radicaux, identiques ou différents, choisis parmi les radicaux alcoyles contenant 1 à 4 atomes de carbone, hydroxy ou alcoxy dont la partie alcoyle contient 1 à 4 atomes de carbone, par hydrogénation sélective au moyen d'hydrogène d'un composé carbonylé de formule générale :

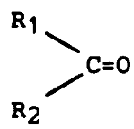
(II)

dans laquelle $R_1$ et $R_2$ sont définis comme ci-dessus, caractérisé en ce que l'on opère en milieu liquide biphasique constitué d'une phase organique et d'une phase essentiellement aqueuse non miscible en présence d'un catalyseur choisi parmi les dérivés du ruthénium associés à un ligand hydrosoluble et les complexes définis du ruthénium avec un ligand hydrosoluble.

Plus particulièrement, la présente invention concerne la préparation d'alcools $\alpha,\beta$-insaturés à partir des composés carbonylés $\alpha,\beta$-insaturés correspondants, c'est-à-dire la préparation des produits de formule générale (I) dans laquelle un au moins des symboles $R_1$ ou $R_2$ contient une double liaison en position $\alpha,\beta$ par rapport à la fonction alcool à partir des composés $\alpha,\beta$-insaturés de formule générale (II) correspondants.

Plus particulièrement, la présente invention concerne la préparation des alcools $\alpha,\beta$-insaturés de formule générale (I) dans laquelle un des symboles $R_1$ ou $R_2$ représente un atome d'hydrogène et l'autre représente un radical aliphatique contenant 1 à 30 atomes de carbone et au moins une double liaison en position $\alpha,\beta$ par rapport à la fonction alcool éventuellement substitué par un ou plusieurs radicaux, identiques ou différents, choisis parmi les radicaux alcoyles contenant 1 à 4 atomes de carbone, hydroxy ou alcoxy dont la partie alcoyle contient 1 à 4 atomes de carbone, ou par un radical alicyclique contenant 5 ou 6 atomes de carbone saturé ou non saturé éventuellement substitué par un ou plusieurs radicaux alcoyles contenant 1 à 4 atomes de carbone, ou par un radical phényle éventuellement substitué, ou bien $R_1$ et $R_2$ forment ensemble un radical alicyclique non saturé éventuellement substitué par un ou plusieurs radicaux alcoyles contenant 1 à 4 atomes de carbone, à partir des composés carbonylés $\alpha,\beta$-insaturés de formule générale (II) correspondants.

Plus particulièrement, la présente invention concerne la préparation du prénol à partir du prénal, du nérol/géraniol à partir du citral, de l'alcool crotylique à partir du crotonaldéhyde ou de l'alcool cinnamique à partir du cinnamaldéhyde.

Alors que l'hydrogénation d'une double liaison carbone-carbone est facilement réalisable en catalyse homogène, la réduction de la fonction carbonyle s'effectue difficilement surtout dans le cas des dérivés carbonylés insaturés.

Il est connu d'effectuer la réduction d'aldéhydes saturés en présence d'un métal du groupe du platine, d'un ligand hydrosoluble et d'un composé amphiphile (EP 61 337). Le brevet européen EP 24648 décrit l'hydrogénation en phase liquide d'aldéhydes insaturés en présence d'un métal supporté et d'une amine tertiaire. Le brevet US 3 968 147 décrit la préparation d'alcools optiquement actifs par hydrogénation de cétones optiquement inactives au moyen d'un catalyseur comprenant un métal du groupe VIII complexé à une phosphine optiquement active.

Il est aussi connu d'utiliser des complexes organo-métalliques à base de rhodium [T. Mizoroki et coll., Bull. Chem. Soc. Japan, 50, 2148 (1977)] ou d'iridium [E. Farnetti et coll., J. Chem. Soc. Chem. Comm., p. 746 (1986)] pour réduire sélectivement l'aldéhyde cinnamique en alcool insaturé correspondant. W. Strohmeier et K. Kolke, J. Organometal. Chem., 193, C63 (1980) ont décrit la réduction de l'aldéhyde crotonique au moyen de complexes du ruthénium, la meilleure sélectivité étant obtenue avec $RuCl_2 [P(C_6H_{11})_3]_2 (CO)_2$. Enfin K. Hotta, J. Mol. Catal., 29, 105-107 (1985) a montré que le citral était transformé en géraniol/nérol par hydrogénation en présence d'un complexe $RuCl_2 [(PPh_3)]_3$ en opérant dans un mélange toluène-éthanol en présence d'un excès d'acide chlorhydrique.

Il a maintenant été trouvé, et c'est ce qui fait l'objet de la présente invention, que les carbonyles insaturés de formule générale (II) peuvent être transformés sélectivement en alcools insaturés de formule générale (I) en opérant dans un milieu biphasique constitué d'une phase organique et d'une phase aqueuse ou essentiellement aqueuse non miscible en présence d'un catalyseur constitué d'un dérivé du ruthénium associé à un ligand hydrosoluble ou d'un complexe du ruthénium avec un ligand hydrosoluble de préférence à une température comprise entre -20 et 200°C et sous une pression comprise entre 1 et 200 bars.

Les ligands hydrosolubles sont plus particulièrement les phosphines hydrosolubles qui sont décrites dans le brevet français FR 76 22824 (2 366 237) et plus spécialement la tri(métasulfophényl) phosphine (TPPTS).

Les dérivés du ruthénium qui conviennent particulièrement bien pour la réalisation du procédé selon l'invention sont choisis parmi les halogénures de ruthénium tels que $RuCl_3, xH_2O$ ; $RuBr_3, xH_2O$ ; $RuI_3, xH_2O$, les oxydes de ruthénium tels que $RuO_2$ ou les sels complexes du ruthénium tels que $(NH_4)_3RuCl_6$, $(NH_4)_2RuCl_5(H_2O)$, $K_2RuCl_5 (H_2O)$, $Ru(NO)(NO_3)_3$, $Ru(NH_3)_6Cl_3$, $K_2Ru(CN)_6$, $[RuCl(NH_3)_5]Cl_2$ ou $K_2[RuCl_5(NO)]$.

Les complexes du ruthénium avec un ligand hydrosoluble sont généralement choisis parmi $RuCl_2(TPPTS)_3$, $H_2Ru(TPPTS)_4$, $HRu(OAc)(TPPTS)_3$, $HRuCl(TPPTS)_3$, $Ru(CO)_2Cl_2(TPPTS)_2$.

Généralement la réaction est effectuée dans l'eau, le produit de formule générale (I) et le produit de formule générale (II) constituant la phase organique. Il est possible cependant de mettre en oeuvre le procédé selon l'invention en présence d'un solvant organique utilisé en quantité telle que le mélange réactionnel est constitué d'une phase organique et d'une phase hydro-organique non miscible.

Les solvants organiques qui conviennent particulièrement bien sont choisis parmi les solvants non miscibles ou peu miscibles à l'eau. Plus particulièrement, les solvants sont choisis parmi les alcools (octanol), les éthers (éther éthylique, tertiobutyléthyléther), les cétones (méthylisobutylcétone), les aldéhydes (benzaldéhyde), les esters (acétate de méthyle, acétate d'éthyle, acétate de butyle) et les hydrocarbures aliphatiques ou aromatiques éventuellement halogénés (hexane, toluène, chlorure de méthylène, chloroforme, chlorobenzène), pris seuls ou en mélange.

L'hydrogénation peut être réalisée à une température comprise entre -20 et 200°C mais de préférence entre 0 et 100°C.

L'hydrogénation peut être réalisée sous une pression de 1 à 200 bars mais de préférence entre 1 et 50 bars.

Généralement, on utilise de 0,001 à 0,01 mole de dérivé du ruthénium par mole de composé carbonylé de formule générale (II) mis en oeuvre.

Lorsque l'on utilise un dérivé du ruthénium associé à un ligand hydrosoluble, on utilise de 0,1 à 200 moles de ligand par rapport au dérivé du ruthénium mis en oeuvre et, de préférence 1 à 100 moles de ligand par rapport au dérivé du ruthénium.

Il est particulièrement avantageux de mettre en oeuvre le procédé selon l'invention dans un milieu tamponné neutre ou légèrement basique.

Le catalyseur ou le système catalytique étant soluble dans l'eau, il peut être facilement séparé, en fin de réaction, par décantation et il peut être ainsi recyclé.

Le procédé selon l'invention permet d'obtenir les alcools insaturés, et plus particulièrement les alcools $\alpha,\beta$-insaturés avec une sélectivité généralement supérieure à 80 %.

Les alcools de formule générale (I) obtenus par la mise en oeuvre du procédé selon l'invention sont des intermédiaires utilisables en synthèse organique. Plus particulièrement, le prénol et le géraniol/nérol sont particulièrement utiles dans la synthèse des vitamines A et E.

Les exemples suivants, donnés à titre non limitatif, montrent comment l'invention peut être mise en pratique.

EXEMPLE 1

Dans une ampoule de verre de 25 cm3, on introduit successivement, sous atmosphère d'argon $10^{-4}$ mole de $RuCl_3$, $3H_2O$, 4 x $10^{-4}$ mole de TPPTS, 7 g d'eau distillée, préalablement désaérée par barbotage d'argon pendant 15 minutes, et 20 x $10^{-4}$ mole de prénal.

L'ampoule est placée dans un autoclave de 125 cm3. On purge 3 fois avec de l'hydrogène puis on établit une pression de 20 bars d'hydrogène et fixe la température à 35°C. L'autoclave est agité par secousses.

Après 3 heures, l'analyse du mélange réactionnel par chromatographie en phase gazeuse montre que :

– le taux de transformation du prénal est de 31 %

– la répartition des produits d'hydrogénation est la suivante :

. prénol : 25 %
. alcool isoamylique : 0,5 %
. isovaléraldéhyde : 0,2 %
. produits non dosés : 4 %

La sélectivité en prénol est de 80 %.

EXEMPLE 2

On opère comme dans l'exemple 1 mais en utilisant 8,5 x $10^{-5}$ mole de $RuCl_2(TPPTS)_3$, 6 g d'eau désaérée et 20 x $10^{-3}$ mole de prénal.

Après 3 heures d'agitation à une température de 35°C et sous une pression d'hydrogène de 32 bars, l'analyse du mélange réactionnel par chromatographie en phase gazeuse montre que :

– le taux de transformation du prénal est de 98 %

– la répartition des produits d'hydrogénation est la suivante :

. prénol : 82 %
. alcool isoamylique : 11 %
. produits non dosés : 5 %

La sélectivité en prénol est de 83 %.

EXEMPLES 3 A 7

On opère comme dans l'exemple 1 en utilisant $10^{-4}$ mole de $RuCl_3$, $3H_2O$, 4 x $10^{-4}$ mole de TPPTS, 20 x $10^{-3}$ mole de prénal, 5 cm3 d'eau désaérée et 5 cm3 d'un solvant non miscible.

On opère sous une pression d'hydrogène de 20 bars à 35°C.

Les résultats sont rassemblés dans le tableau 1.

TABLEAU 1

| Exemple | Solvant | Temps de réaction | Taux de transformation du prénal | Rendement en prénol |
|---|---|---|---|---|
| 3 | hexane | 2 h 30 | 97,5 | 83 |
| 4 | toluène | 1 h 10 | 96 | 97 |
| 5 | acétate d'éthyle | 1 h 15 | 93 | 90 |
| 6 | chloroforme | 1 h 10 | 84 | 96 |
| 7 | chlorure de méthylène | 1 h 30 | 94 | 88 |

EXEMPLE 8

On opère comme dans l'exemple 1 mais en utilisant $10^{-4}$ mole de $RuCl_3$, $3H_2O$, 4 x $10^{-4}$ mole de TPPTS, 5 cm3 d'une solution tampon à pH = 7, 5 cm3 de toluène et 20 x $10^{-3}$ mole de prénal.

On opère sous une pression d'hydrogène de 20 bars à 35°C.

Après 1 heure 8 minutes, l'analyse du mélange réactionnel en phase gazeuse montre que :

– le taux de transformation du prénal est de 99 %

– la répartition des produits d'hydrogénation est la suivante :

. prénol : 98 %
. alcool isoamylique : 0,5 %
. isovaléraldéhyde : non détectable

Le rendement en prénol est de 99 %.

EXEMPLE 9

Dans une ampoule de verre de 25 cm3, on introduit successivement, sous atmosphère d'argon :

- $RuCl_3$, $3H_2O$ 0,032 g ($1,2 \times 10^{-4}$ mole)
- TPPTS 0,32 g ($5,2 \times 10^{-4}$ mole)

On introduit ensuite, au moyen d'un tube de transfert, 4,9 g de toluène et 4,9 g d'eau préalablement désaérés par bullage d'argon pendant 15 minutes. On ajoute alors, au moyen d'une seringue, $21 \times 10^{-3}$ mole de prénal.

L'ampoule est placée dans un autoclave de 125 cm3 en acier inoxydable. On purge 3 fois avec de l'hydrogène puis on établit la pression d'hydrogène à 20 bars et la température à 35°C. L'autoclave est agité par secousses.

Après 75 minutes, l'analyse du mélange réactionnel par chromatographie en phase gazeuse montre que :

– le taux de transformation du prénal est de 98 %
– la répartition des produits d'hydrogénation est la suivante :

. prénol : 94 %
. alcool isoamylique : 1,9 %
. isovaléraldéhyde : non détectable
. alcool t.amylique : voisin de 2 %

La sélectivité est de 97 %.

Après hydrogénation, l'ampoule est sortie de l'autoclave et placée sous atmosphère inerte. La phase organique incolore est séparée par décantation. La phase aqueuse rouge est lavée 2 fois sous atmosphère d'argon avec du toluène dégazé avant d'être recyclée.

Le tableau suivant donne les résultats obtenus après chaque recyclage :

| N° de recyclage | Durée de l'hydrogénation (en minutes) | TT | RT |
|---|---|---|---|
| 1 | 65 | 97 | 95 |
| 2 | 65 | 97 | 92 |
| 3 | 65 | 98 | 92 |
| 4 | 70 | 82 | 97 |

TT : taux de transformation du prénal

RT : rendement en prénol par rapport au prénal transformé

L'activité moyenne du catalyseur est de 160 moles de prénal transformé par mole de catalyseur et par heure.

EXEMPLE 10

Dans une ampoule de verre, on introduit successivement, sous atmosphère d'argon :

- $RuCl_3$, $3H_2O$ $1,8 \times 10^{-4}$ mole
- TPPTS $5,5 \times 10^{-4}$ mole
- toluène 4,4 g
- mélange tampon à pH = 7 5 g
- citral $9,06 \times 10^{-3}$ mole

L'ampoule est introduite dans un autoclave de 125 cm3. On purge 3 fois à l'hydrogène avant d'établir la pression d'hydrogène à 50 bars et la température à 50°C. L'autoclave est agité par secousses.

Après 15 heures, l'analyse par chromatographie en phase vapeur montre que :
– le taux de transformation du citral est de 96,4 %
– la répartition des produits d'hydrogénation est la suivante :
. nérol/géraniol : 94,2 %
. citronellol : 2 %
. citronellal : non détectable
. tétrahydrogéraniol : non détectable
La sélectivité en nérol/géraniol est de 97,7 %.

EXEMPLE 11

On opère comme dans l'exemple 10, mais en utilisant :
- $RuCl_3$, $3H_2O$ $10^{-4}$ mole
- TPPTS $4,72 \times 10^{-4}$ mole
- hexane 3,4 g
- mélange tampon à pH = 7 5 g
- crotonaldéhyde $24 \times 10^{-3}$ mole

On opère sous une pression d'hydrogène de 20 bars à 35°C.
Après 4 heures, l'analyse par chromatographie en phase vapeur montre que :
– le taux de transformation du crotonaldéhyde est de 95,5 %
– la répartition des produits d'hydrogénation est la suivante :
. alcool crotylique : 92,7 %
. butanal : 1 %
. butanol 1,7 %
La sélectivité en alcool crotylique est de 97 %.

EXEMPLE 12

On opère comme dans l'exemple 10, mais en utilisant :
- $RuCl_3$, $3H_2O$ $9,8 \times 10^{-5}$ mole
- TPPTS $4,4 \times 10^{-4}$ mole
- toluène 4,3 g
- milieu tampon à pH = 7 4,8 g
- aldéhyde cinnamique $13,2 \times 10^{-3}$ mole

On opère sous une pression d'hydrogène de 20 bars à 35°C.
Après 3 heures, l'analyse par chromatographie en phase vapeur montre que :
– le taux de transformation est de 98,8 %
– la répartition des produits d'hydrogénation est la suivante :
. alcool cinnamique : 98,5 %
. phényl-3 propanal : non détectable
. phényl-3 propanol : non détectable
La sélectivité en alcool cinnamique est de 99,5 %.

EXEMPLE 13

Dans un ballon de 250 cm3, on introduit $5 \times 10^{-4}$ mole de chlorure de ruthénium ($RuCl_3$) et $17,5 \times 10^{-3}$ mole de TPPTS puis on ajoute, sous atmosphère inerte, 100 cm3 de solution tampon à pH = 7. La solution obtenue, de couleur vert foncé, est transférée à l'aide d'une seringue dans un réacteur SOTELEM de 300 cm3 préalablement purgé à l'azote. Après fermeture du réacteur, on purge à l'azote puis à l'hydrogène et ajuste la pression d'hydrogène à 20 bars et la température à 50°C. On agite à une vitesse de 1500 tours/minute pendant 1 heure. Après arrêt de l'agitation et refroidissement à une température voisine de 20°C, le réacteur est dégazé avec précaution puis purgé à l'azote.

Sous courant d'azote, on introduit 50 cm3 de prénal. Le réacteur est purgé à l'azote puis à l'hydrogène. On fixe la pression d'hydrogène à 20 bars et la température à 50°C. L'agitation est réglée à la vitesse de 1500 tours/minute. L'hydrogénation dure 30 minutes.

Après refroidissement jusqu'à une température de 20°C, le réacteur est dégazé avec précaution. Le mélange réactionnel est versé dans une ampoule à décanter. La phase aqueuse est extraite 3 fois avec 25 cm3 de chlorure de méthylène.

L'analyse des phases organiques réunies par chromatographie en phase gazeuse montre que :

– le taux de transformation du prénal est de 100 %

– la répartition des produits d'hydrogénation est la suivante :

| | |
|---|---|
| . prénol | : 99 % |
| . isoprénol | : 0,6 % |
| . alcool isoamylique | : 0,2 % |
| . produits non dosés | : 0,2 % |

## Revendications

1. Procédé de préparation d'alcools insaturés de formule générale :

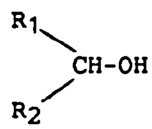

dans laquelle $R_1$ et $R_2$, identiques ou différents, représentent un atome d'hydrogène ou un radical aliphatique saturé ou non saturé, éventuellement substitué par un radical alicyclique saturé ou non saturé ou par un radical aromatique, ou un radical alicyclique saturé ou non saturé ou un radical aromatique, ou bien $R_1$ et $R_2$ forment ensemble un radical alicyclique non saturé, étant entendu que :

– $R_1$ et $R_2$ ne peuvent pas représenter simultanément un atome d'hydrogène,

– l'un au moins des radicaux $R_1$ ou $R_2$ contient une double liaison éthylénique,

– les radicaux aliphatiques, alicycliques ou aromatiques peuvent être éventuellement substitués par un ou plusieurs radicaux, identiques ou différents, choisis parmi les radicaux alcoyles contenant 1 à 4 atomes de carbone, hydroxy ou alcoxy dont la partie alcoyle contient 1 à 4 atomes de carbone, par hydrogénation sélective au moyen d'hydrogène d'un composé carbonylé de formule générale :

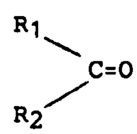

dans laquelle $R_1$ et $R_2$ sont définis comme précédemment, caractérisé en ce que l'on opère dans un milieu biphasique constitué d'une phase organique et d'une phase essentiellement aqueuse non miscible en présence d'un catalyseur à base de ruthénium choisi parmi les dérivés du ruthénium associés à un ligand hydrosoluble et les complexes définis du ruthénium avec un ligand hydrosoluble.

2. Procédé selon la revendication 1 caractérisé en ce que les dérivés du ruthénium associés à un ligand hydrosoluble sont choisis parmi les sels minéraux ou organiques, les oxydes, les hydrures et les sels complexes de ruthénium.

3. Procédé selon la revendication 2 caractérisé en ce que les dérivés du ruthénium associés à un ligand hydrosoluble sont choisis parmi $RuCl_3$, $xH_2O$ ; $RuBr_3$, $xH_2O$ ; $RuI_3$, $xH_2O$ ; $RuO_2$, $(NH_4)_3RuCl_6$, $(NH_4)_2RuCl_5(H_2O)$ ; $K_2RuCl_5$ $(H_2O)$, $Ru(NO)(NO_3)_3$ ; $Ru(NH_3)_6Cl_3$ ; $K_2Ru(CN)_6$ ; $[RuCl(NH_3)_5]Cl_2$ ou $K_2[RuCl_5(NO)]$.

4. Procédé selon la revendication 1 caractérisé en ce que les complexes définis du ruthénium avec un ligand hydrosoluble sont choisis parmi $RuCl_2L_3$, $H_2RuL_4$, $HRu(OAc)L_3$, $HRuClL_3$ ou $Ru(CO)_2$ $Cl_2L_4$, L représentant un ligand hydrosoluble.

5. Procédé selon l'une des revendications 1 à 4 caractérisé en ce que le ligand hydrosoluble est choisi parmi les phosphines solubles dans l'eau.

6. Procédé selon la revendication 5 caractérisé en ce que les phosphines solubles dans l'eau sont choisies parmi les phénylphosphines sulfonées.

7. Procédé selon la revendication 6 caractérisé en ce que la phénylphosphine sulfonée est la tri(métasulfophényl) phosphine (TPPTS).

8. Procédé selon la revendication 1 caractérisé en ce que l'on opère en outre en présence d'un solvant organique en quantité suffisante pour maintenir l'existence d'une phase organique et d'une phase essentiellement aqueuse non miscible.

9. Procédé selon la revendication 8 caractérisé en ce que le solvant organique est choisi parmi les alcools, les éthers, les aldéhydes, les cétones, les esters et les hydrocarbures aliphatiques ou aromatiques éventuellement halogénés utilisés seuls ou en mélange.

10. Procédé selon l'une des revendications 1 à 9 caractérisé en ce que l'on opère à une température comprise entre -20 et 200°C.

11. Procédé selon la revendication 10 caractérisé en ce que l'on opère à une température comprise entre 0 et 100°C.

12. Procédé selon l'une des revendications 1 à 9 caractérisé en ce que l'on opère sous une pression comprise entre 1 et 200 bars.

13. Procédé selon la revendication 12 caractérisé en ce que l'on opère sous une pression comprise entre 1 et 50 bars.

14. Procédé selon l'une des revendications 1 à 13 caractérisé en ce que l'on hydrogène sélectivement le prénal en prénol, le citral en géraniol/nérol, le crotonaldéhyde en alcool crotylique et le cinnamaldéhyde en alcool cinnamique.

**Patentansprüche**

1. Verfahren zur Herstellung ungesättigter Alkohole der allgemeinen Formel:

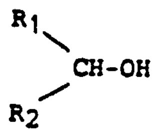

in welcher $R_1$ und $R_2$ unabhängig voneinander ein Wasserstoffatom oder einen gesättigten oder ungesättigten aliphatischen Rest, gegebenenfalls substituiert durch einen gesättigten oder ungesättigten alicyclischen Rest oder durch einen aromatischen Rest, oder einen gesättigten oder ungesättigten alicyclischen Rest oder einen aromatischen Rest darstellen oder $R_1$ und $R_2$ zusammen einen ungesättigten alicyclischen Rest bilden, wobei selbstverständlich:

– $R_1$ und $R_2$ nicht gleichzeitig ein Wasserstoffatom darstellen können,
– zumindest einer der Reste $R_1$ oder $R_2$ eine äthylenische Doppelbindung enthält,
– die aliphatischen, alicyclischen oder aromatischen Reste gegebenenfalls durch einen oder mehrere gleiche oder verschiedene Reste, ausgewählt aus den Alkylresten mit 1 bis 4 Kohlenstoffatomen, den Hydroxyresten oder Alkoxyresten, deren Alkylteil 1 bis 4 Kohlenstoffatome enthält, substituiert sein können, durch selektive hydrierung einer Carbonylverbindung der allgemeinen Formel:

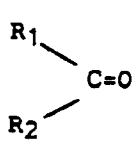

in welcher $R_1$ und $R_2$ die obige Bedeutung haben, dadurch gekennzeichnet, daß man in einem zweiphasigen Milieu, bestehend aus einer organischen Phase und einer im wesentlichen nicht mischbaren wässerigen Phase, in Gegenwart eines Katalysators auf Rutheniumbasis, ausgewählt aus den mit einem wasserlöslichen Liganden assoziierten Rutheniumderivaten und den definierten Komplexen von Ruthenium mit einem wasserlöslichen Liganden, arbeitet.

2. Verfahren nach Anspruch 1, dadurch gekennzeichnet, daß die mit einem wasserlöslichen Liganden assoziierten Rutheniumderivate ausgewählt sind aus den anorganischen oder organischen Salzen, den Oxiden, den Hydriden und den Komplexsalzen von Ruthenium.

3. Verfahren nach Anspruch 2, dadurch gekennzeichnet, daß daß die mit einem wasserlöslichen Liganden assoziierten Rutheniumderivate ausgewählt sind aus $RuCl_3$, $xH_2O$; $RuBr_3$, $xH_2O$; $RuJ_3$, $xH_2O$; $RuO_2$, $(NH_4)_3RuCl_6$, $(NH_4)_2RuCl_5(H_2O)$; $K_2RuCl_5$ $(H_2O)$, $Ru(NO)(NO_3)_3$; $Ru(NH_3)_6Cl_3$; $K_2Ru(CN)_6$; $[RuCl(NH_3)_5]Cl_2$ oder $K_2[RuCl_5(NO)]$.

4. Verfahren nach Anspruch 1, dadurch gekennzeichnet, daß die definierten Komplexe von Ruthenium mit

einem wasserlöslichen Liganden ausgewählt sind aus $RuCl_2L_3$, $H_2RuL_4$, $HRu(OAc)L_3$, $HRuClL_3$ oder $Ru(CO)_2Cl_2L_4$, wobei L einen wasserlöslichen Liganden darstellt.

5. Verfahren nach einem der Ansprüche 1 bis 4, dadurch gekennzeichnet, daß daß der wasserlösliche Ligand ausgewählt ist aus den wasserlöslichen Phosphinen.

6. Verfahren nach Anspruch 5, dadurch gekennzeichnet, daß die wasserlöslichen Phosphine ausgewählt sind aus den sulfonierten Phenylphosphinen.

7. Verfahren nach Anspruch 6, dadurch gekennzeichnet, daß das sulfonierte Phenylphosphin Tri(metasulfophenyl)phosphin (TPPTS) ist.

8. Verfahren nach Anspruch 1, dadurch gekennzeichnet, daß man außerdem in Gegenwart eines organischen Lösungsmittels in einer Menge arbeitet, die ausreicht, um den Bestand einer organischen Phase und einer im wesentlichen wässerigen nicht mischbaren Phase aufrechtzuerhalten.

9. Verfahren nach Anspruch 8, dadurch gekennzeichnet, daß das organische Lösungsmittel ausgewählt ist aus den Alkoholen, den Äthern, den Aldehyden, den Ketonen, den Estern und den gegebenenfalls halogenierten aliphatischen oder aromatischen Kohlenwasserstoffen, allein oder in Mischung verwendet.

10. Verfahren nach einem der Ansprüche 1 bis 9, dadurch gekennzeichnet, daß man bei einer Temperatur zwischen -20 und 200°C arbeitet.

11. Verfahren nach Anspruch 10, dadurch gekennzeichnet, daß man bei einer Temperatur zwischen 0 und 100°C arbeitet.

12. Verfahren nach einem der Ansprüche 1 bis 9, dadurch gekennzeichnet, daß man unter einem Druck zwischen 1 und 200 bar arbeitet.

13. Verfahren nach Anspruch 12, dadurch gekennzeichnet, daß man unter einem Druck zwischen 1 und 50 bar arbeitet.

14. Verfahren nach einem der Ansprüche 1 bis 13, dadurch gekennzeichnet, daß man selektiv hydriert: Prenal zu Prenol, Citral zu Geraniol/Nerol, Krotonaldehyd zu Krotylalkohol und Zimtaldehyd zu Zimtalkohol.

## Claims

1. Process for the preparation of unsaturated alcohols of the general formula:

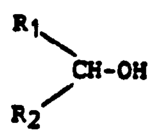

in which $R_1$ and $R_2$, which are identical or different, represent a hydrogen atom, a saturated or unsaturated aliphatic radical optionally substituted by a saturated or unsaturated alicyclic radical or by an aromatic radical, a saturated or unsaturated alicyclic radical or an aromatic radical, or alternatively $R_1$ and $R_2$ together form an unsaturated alicyclic radical, it being understood that:

- $R_1$ and $R_2$ cannot simultaneously represent a hydrogen atom,
- at least one of the radicals $R_1$ and $R_2$ contains an ethylenic double bond, and
- the aliphatic, alicyclic or aromatic radicals can optionally be substituted by one or more identical or different radicals selected from alkyl radicals containing 1 to 4 carbon atoms, hydroxyl radicals and alkoxy radicals of which the alkyl part contains 1 to 4 carbon atoms, by selective hydrogenation, by means of hydrogen, of a carbonyl compound of the general formula:

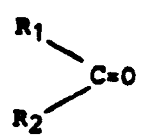

in which $R_1$ and $R_2$ are as defined above, characterised in that the reaction is carried out in a two-phase medium consisting of an organic phase and an essentially aqueous, immiscible phase, in the presence of a ruthenium-based catalyst selected from ruthenium derivatives associated with a water-soluble ligand and defined complexes of ruthenium with a water-soluble ligand.

2. Process according to claim 1, characterised in that the ruthenium derivatives associated with a water-soluble ligand are selected from the inorganic or organic salts, oxides, hydrides and complex salts of ruthenium.

3. Process according to claim 2, characterised in that the ruthenium derivatives associated with a water-soluble ligand are selected from $RuCl_3$. $xH_2O$, $RuBr_3$. $xH_2O$, $RuI_3$. $xH_2O$, $RuO_2$, $(NH_4)_3RuCl_6$, $(NH_4)_2RuCl_5(H_2O)$, $K_2RuCl_5$ $(H_2O)$, $Ru(NO)(NO_3)_3$, $Ru(NH_3)_6Cl_3$, $K_2Ru(CN)_6$, $[RuCl(NH_3)_5]Cl_2$ and $K_2(RuCl_5(NO))$].

4. Process according to claim 1, characterised in that the defined complexes of ruthenium with a water-soluble ligand are selected from $RuCl_2L_3$, $H_2RuL_4$, $HRu(OAc)L_3$, $HRuClL_3$ and $Ru(CO)_2Cl_2L_4$, L representing a water-soluble ligand.

5. Process according to any one of claims 1 to 4, characterised in that the water-soluble ligand is selected from water-soluble phosphines.

6. Process according to claim 5, characterised in that the water-soluble phosphines are selected from sulphonated phenylphosphines.

7. Process according to claim 6, characterised in that the sulphonated phenylphosphine is tri(meta-sulphophenyl)phosphine (TPPTS).

8. Process according to claim 1, characterised in that the reaction is additionally carried out in the presence of an organic solvent in a sufficient amount to maintain the existence of an organic phase and an essentially aqueous, immiscible phase.

9. Process according to claim 8, characterised in that the organic solvent is selected from alcohols, ethers, aldehydes, ketones, esters and optionally halogenated aliphatic or aromatic hydrocarbons, used on their own or as a mixture.

10. Process according to one of claims 1 to 9, characterised in that the reaction is carried out at a temperature of between -20 and 200°C.

11. Process according to claim 10, characterised in that the reaction is carried out at a temperature of between 0 and 100°C.

12. Process according to one of claims 1 to 9, characterised in that the reaction is carried out under a pressure of between 1 and 200 bars.

13. Process according to claim 12, characterised in that the reaction is carried out under a pressure of between 1 and 50 bars.

14. Process according to one of claims 1 to 13, characterised in that prenal is hydrogenated selectively to prenol, citral to geraniol/nerol, crotonaldehyde to crotyl alcohol and cinnamaldehyde to cinnamyl alcohol.